# EUROPEAN PATENT SPECIFICATION

(11) **EP 2 077 891 B1**
(45) Date of publication and mention of the grant of the patent: **19.05.2010**
(21) Application number: 07748807.0
(22) Date of filing: 30.05.2007
(51) Int. Cl.: A61N 1/16

(54) **DEVICE FOR PROTECTION AGAINST EXPOSURE TO ENERGY**
VORRICHTUNG ZUM SCHUTZ VOR AUSSETZUNG GEGEN ENERGIE
DISPOSITIF DE PROTECTION CONTRE UNE EXPOSITION À L'ÉNERGIE

(30) Priority: 16.10.2006 UA 2006010886
(43) Date of publication of application: 15.07.2009
(73) Proprietor: Volkov, Aleksey Yevgenyevich, Kharkov 61006 (UA)
(72) Inventor: Volkov, Aleksey Yevgenyevich, Kharkov 61006 (UA)
(74) Representative: Ripamonti, Enrico
(86) International application number: PCT/UA2007/000035
(87) International publication number: WO 2008/048196

(56) References cited:
- WO-A-2004/089053
- DE-A1- 3 631 354
- DE-U1- 9 302 948
- RU-C1- 2 259 215

## Description

### FIELD OF THE INVENTION

The invention relates to the protection of biological objects such as humans, animals, *etc*. against exposure to electromagnetic radiations, which accompany the operation of electric and power devices, and may be practiced at home and in everyday life.

### DESCRIPTION OF THE PRIOR ART

In the process of his or her everyday life, a human is continuously in a common energy and information field that is generated as a result of the addition of fields of various radiation sources, *viz.,* electromagnetic radiation, cosmic radiation, geomagnetic radiation, *etc.* An energy and information frequency spectrum of living matter affecting both physical and physiological process by means of the common energy and information field has been determined using mathematical simulation methods. To protect biological objects (for instance, humans and animals), devices were created to protect against exposure to energy. Such devices belong to the class of apparatuses used to reduce electromagnetic radiation, which accompanies the operation of electric and power devices, and to reduce the exposure of biological objects to electromagnetic radiations. The effectiveness of devices of such type has been proved experimentally.

Known in the prior art is a device for protection against exposure to energy comprising a dielectric plate with two effective surfaces and metal applicators arranged thereon. The first effective surface of the dielectric plate includes applicators in the shape of three-, four-, five-, six- and eight-beam stars arranged thereon, and the second effective surface of the dielectric plate includes two applicators arranged oppositely thereon, each of which is made in the shape of a saw-toothed polygon (Russian Patent No. 2259215, IPC A61 N 1/16, published on August 27, 2005). This device is also provided with an applicator in the shape of a seven-beam star. This prior art device features an additional dielectric plate with applicators in shape of the spiral of Archimedes arranged thereon.

A disadvantage of the prior art device for protection against exposure to energy is that it is of a low effectiveness. This is due to that it fails to ensure a necessary polarization degree of electromagnetic radiation created by an external radiation source this resulting in an insufficient reduction in the external electromagnetic radiation and in an insufficient absorption of the external electromagnetic radiation to which a biological object is exposed. The largest portion of electromagnetic radiation harmful for humans is in a high-frequency spectrum, which spectrum is neutralized, in the prior art device, by means of one eight-beam applicator, the power of which is insufficient this having been proved by experimental investigations. Furthermore, this device is provided with metal ties magnetized in one direction. It should be pointed out, however, that these ties are demagnetized when the device is in use this decreasing the effectiveness of its functioning. It should be also mentioned that, due to the employment of the additional dielectric plate in the device and, as a consequence, increase in the spatial volume thereof, undesired energy fluxes are generated that decrease the effectiveness of functioning of the device. Another essential disadvantage of the prior art device is that the connection of applicators to each other results in increase in stray currents and, as a consequence, in the unbalance of effects of the applicators on shielding properties of the prior art device, that is to say, its properties to reduce electromagnetic radiations, which accompany the operation of electric and power devices.

The most similar to the device in accordance with this invention is a device for protection against exposure to energy comprising a dielectric plate with two effective surfaces and metal applicators arranged thereon. The first effective surface of the dielectric plate includes applicators in the shape of three-, four-, five-, six- and eight-beam stars arranged thereon, and the second effective surface of the dielectric plate includes two applicators arranged oppositely thereon, each of which is made in the shape of a saw-toothed polygon (Russian Patent No. 2259215, IPC A 61 N 1/16, published on August 27, 2005). This device is also provided with an applicator in the shape of a seven-beam star, which is arranged on the first effective surface of the dielectric plate. This prior art device is **characterized in that** the second effective surface of the dielectric plate includes, in addition to two saw-toothed applicators arranged oppositely thereon, two applicators in the shape of a triangle and an eight-beam star additionally arranged thereon. Another feature of this prior art device is that the applicators arranged on the first effective surface of the dielectric plate and made in the shape of the three-, four-, six- and eight-beam stars contact to each other, and two applicators arranged on the first effective surface of the dielectric plate and made in the shape of the five- and seven-beam stars also contact to each other. On the second effective surface of the dielectric plate, all the applicators contact to each other.

A disadvantage of the prior art device for protection against exposure to energy is that it is of a low effectiveness. This is due to that it fails to ensure a necessary polarization degree of electromagnetic radiation created by an external radiation source this resulting in an insufficient reduction in the external electromagnetic radiation and in an insufficient absorption of the external electromagnetic radiation to which a biological object is exposed. The use of only six applicators arranged on the first effective surface of the dielectric plate, one of which is made in the shape of a seven-beam star, does not ensure an intensive polarization of electromagnetic radiation. The use of an eight-beam applicator along with the seven-beam applicator on the first effective surface of the dielectric plate does not ensure an effective harmonization of polarization vectors and reduces the operating frequency range of the prior art device this being a cause of a low effectiveness of its functioning. The connection of applicators to each other results in increase in stray currents and, as a consequence, in the unbalance of effects of the applicators on shielding properties of the prior art device this decreasing the effectiveness of its functioning.

The largest portion of electromagnetic radiation harmful for humans is in a high-frequency spectrum, which portion is neutralized, in the prior art device, by means of one eight-beam applicator, the power of which is insufficient this having been proved by experimental investigations. The shape and configuration of the applicators arranged on the second effective surface of the dielectric plate do not allow both uniform and intensive reduction in a high-frequency electromagnetic radiation to be achieved.

### DISCLOSURE OF THE INVENTION

Accordingly, the present invention has as its object to provide a device for the protection of biological objects against exposure to energy, characterized by improved capabilities, that is to say, capabilities of reducing effectively electromagnetic radiations, which accompany the operation of electric and power devices.

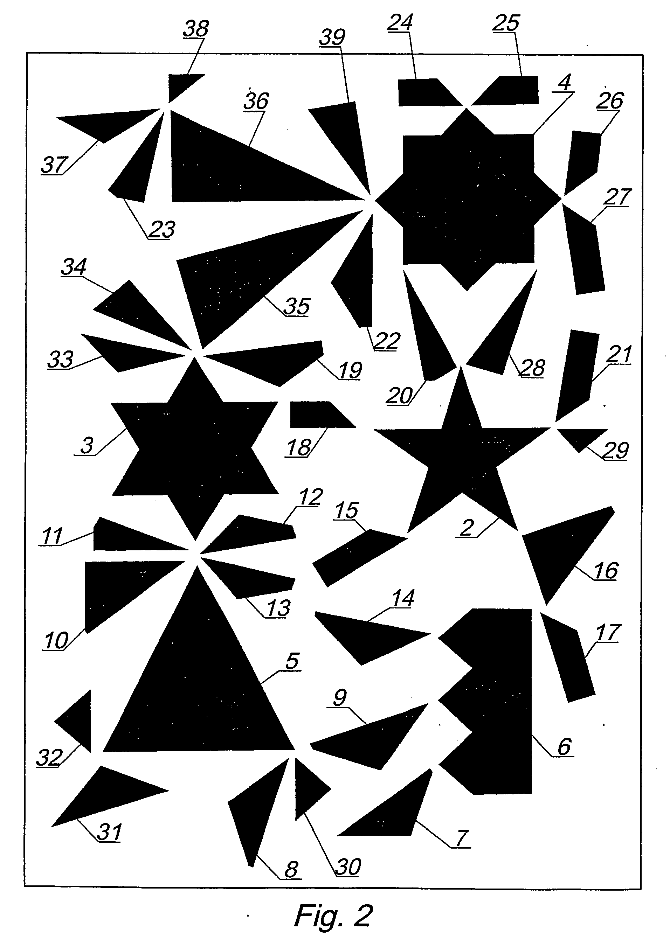

The object of the invention is achieved in that, in the prior art device for protection against exposure to energy comprising a dielectric plate (1), an effective surface whereof includes metal applicators in the shape of a five-beam star (2), a six-beam star (3) and an eight-beam star (4), a triangle (5), and an n-beam saw-toothed polygon (6) arranged thereon, in accordance with the invention, the effective surface of the dielectric plate (1) is provided with twenty-one additional metal applicators (7) to (27) in the shape of quadrangles and twelve additional metal applicators (28) to (39) in the shape of triangles all the applicators being arranged on the effective surface of the dielectric plate 1 as can be seen in Fig. 2.

In at least one embodiment of the device for protection against exposure to energy in accordance with this, invention, the dielectric plate (1) is coated with a dielectric material layer (40).

This invention provides reduction in (increase in the level of dumping) an electromagnetic radiation, which accompanies the operation of electric and power devices, due to increase in polarization degree thereof with the aid of the device in accordance with the invention. This becomes possible thanks to providing the device in accordance with the invention with thirty-three additional metal applicators (7) to (39), of which twenty-one additional metal applicators (7) to (27) have the shape of quadrangles and twelve additional metal applicators (28) to (39) has the shape of triangles. The arrangement of additional metal applicators (7) to (39) on the effective surface of the dielectric plate (1), as illustrated in Fig. 2, makes it possible to combine optimally their effect with that of the applicators (2) to (6) and to amplify the latter this enabling the maximum polarization of and reduction in an external electromagnetic radiation to be ensured this in turn resulting in the effectiveness of functioning of the device in accordance with this invention.

Thanks to the use of the aggregate of the applicators (3), (5), (8), (9), (10), (11), (12), (13), (30), (31), (32) with the chosen orientation, as illustrated in Fig. 2, of the additional applicators (8), (9), (10), (11), (12), (13) made in the shape of quadrangles and the additional applicators (30), (31), (32) made in the shape of triangles, the production of a first area of an elevated strength of a field induced in the device in accordance with the invention is ensured, which field interacts with the first harmonic component of the external electromagnetic radiation and reduces the same.

Thanks to the use of the aggregate of the applicators (4), (22), (35), (36), (39) with the chosen orientation of beams, as illustrated in Fig. 2, of the additional applicators (35), (36), (39) made in the shape of triangles, and of the additional applicator (22) made in the shape of a quadrangle, the production of a second area of an elevated strength of the field induced in the device in accordance with the invention is ensured, which field suppresses the second harmonic component of the external electromagnetic radiation.

Thanks to the use of the aggregate of the applicators (3), (33), (34), (35), (19) with the chosen orientation of beams of the additional applicators (33), (34), (35) made in the shape of triangles, and of the additional applicator (19) made in the shape of a quadrangle, the production of a third area of an elevated strength of the field induced in the device in accordance with the invention is ensured, which field interacts with the third harmonic component of the external electromagnetic radiation and suppresses the same.

Thanks to the use of the aggregate of the applicators (23), (36), (37), (38) with the chosen orientation of beams of the additional applicators (36), (37), (38) made in the shape of triangles, and of the additional applicator (23) made in the shape of a quadrangle, the production of a forth area of an elevated strength of the field induced in the device in accordance with the invention is ensured, which field interacts, respectively, with the forth harmonic component of the external electromagnetic radiation and reduces the same.

Such combined effect of the device in accordance with this invention on the first, second, third, and forth harmonic components of the electromagnetic field ensures the de-structuring of the external electromagnetic radiation with the result of achieving a high level of its reduction this improving the effectiveness of functioning of the device in accordance with this invention.

The aggregate of the applicators (2), (15), (16), (18), (20), (21), (28), (29) with the chosen orientation of beams of the additional applicators (28), (29) made in the shape of triangles, and of the additional applicators (15), (16), (18), (20), (21) made in the shape of quadrangles produces a local area of lamination of the field induced in the device in accordance with the invention, which area absorbs higher harmonic components of the external electromagnetic radiation field.

The aggregate of the applicators (4), (6), (7), (14), (17), (24), (25), (26), (27) with the chosen orientation of beams of the additional applicators (7), (14), (17), (24), (25), (26), (27) made in the shape of quadrangles stabilizes the level of the total field induced in the device in accordance with the invention, the vector of which filed is directed opposite to that of the external electromagnetic radiation field.

The aggregate arrangement of the applicators (2) to (39) on the effective surface of the dielectric plate (1) in accordance with the drawing (Fig. 2) is *sine qua non* of achieving the engineering result claimed by the applicant. It was found, as result of testing, that any other arrangement of the applicators (2) to (39) on the effective surface of the dielectric plate (1) would result in the de-structuring of the field induced in the device in accordance with this invention and would not ensure an effective reduction of electromagnetic radiation.

Coating the dielectric plate (1) with the dielectric material layer (40) prevents the applicators from being contacted to atmospheric air and their microstructure from being damaged in order to preserve predetermined characteristics of the device in accordance with this invention.

It was found, as result of investigations, that the device in accordance with this invention reduces effectively the exposure of biological objects, in particular, humans, to electromagnetic radiations.

### BRIEF DESCRIPTION OF THE DRAWINGS

Fig. 1 is a general view a device in accordance with this invention.
Fig. 2 is a view of an effective surface of a dielectric plate.

### DESCRIPTION OF THE PREFERRED EMBODIMENT

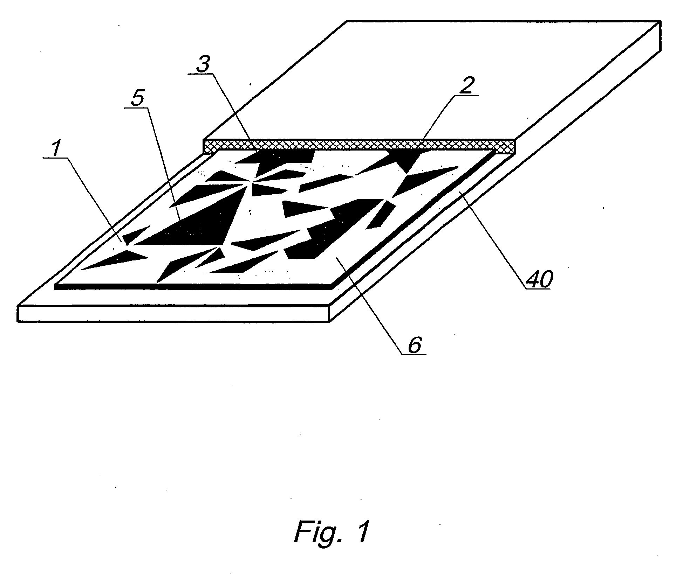

Referring first to Fig. 1, a device for protection against exposure to energy comprises a dielectric plate 1 that includes an effective surface located on one side of the dielectric plate 1. The effective surface of the dielectric plate 1 includes metal applicators arranged thereon (see Fig. 2): an applicator 2 made in the shape of a five-beam star; an applicator 3 made in the shape of a six-beam star; an applicator 4 made in the shape of an eight-beam star; an applicator 5 made in the shape of a triangle; and an applicator 6 made in the shape of an n-beam saw-toothed polygon.

In addition, the effective surface of the dielectric plate 1 is provided with thirty-three additional metal applicators 7 to 39 arranged thereon, of which twenty-one additional metal applicators 7 to 27 have the shape of quadrangles and twelve additional metal applicators 28 to 39 has the shape of triangles.

The general arrangement of the metal applicators 2-39 on the effective surface of the dielectric plate 1 in the exact conformity with the illustration in the drawing (Fig. 2) is *sine qua non* of achieving the engineering result claimed by the applicant.

Referring again to Fig. 1, the dielectric plate 1 is coated with a dielectric material layer 15 which protects the dielectric plate 1 against damages when in use.

The device for protection against exposure to energy in accordance with this invention functions as follows:

The device is placed between an electromagnetic radiation source and a biological object (for instance, a human) to be protected. The applicators 2 to 39 convert this electromagnetic radiation so that external field radiation parameters in the active area of the device approach zero. This conversion occurs thanks to the superposition of the vectors of the external field and those of a field induced in the device. Both magnitude and direction of the induced field vector are added to the external field vector this resulting in a significant reduction in the external field and, as a consequence, in an essential decrease in the exposure of the biological object to it. The arrangement of the additional metal applicators 7 to 39 on the effective surface of the dielectric plate 1, as illustrated in Fig. 2, combines optimally their effect with that of the applicators (2) to (6) and amplifies the latter enabling the maximum polarization of and reduction in an external electromagnetic radiation to be ensured.

The device in accordance with this invention may be practiced both under industrial conditions and at home.

## Claims

1. A device for protection against exposure to energy comprising a dielectric plate (1), an effective surface whereof includes metal applicators in the shape of a five-beam star (2), a six-beam star (3) and an eight-beam star (4), a triangle (5), and an n-beam saw-toothed polygon (6) arranged thereon, ***characterized in that*** the effective surface of the dielectric plate (1) is provided with twenty-one additional metal applicators (7) to (27) in the shape of quadrangles and twelve additional metal applicators (28) to (39) in the shape of triangles all the applicators being arranged on the effective surface of the dielectric plate 1 as can be seen in Fig. 2.

2. The device for protection against exposure to energy of Claim 1, ***characterized in that*** *the* dielectric plate (1) is coated with a dielectric material layer (40).

## Patentansprüche

1. Vorrichtung zum Schutz vor Energieaussetzung, umfassend eine dielektrische Platte (1), wobei eine wirksame Oberfläche derselben darauf angeordnete Metallapplikatoren in der Form eines fünf-strahligen Sterns (2), eines sechs-strahligen Sterns (3) und eines acht-strahligen Sterns (4), eines Dreiecks (5) und eines n-strahligen Sägezahn-Polygons (6) umfasst, **dadurch gekennzeichnet, dass** die wirksame Oberfläche der dielektrischen Platte (1) mit einundzwanzig zusätzlichen Metallapplikatoren (7) bis (27) in der Form von Vierecken und zwölf zusätzlichen Metallapplikatoren (28) bis (39) in der Form von Dreiecken versehen ist, wobei alle Applikatoren wie in Fig. 2 ersichtlich an der wirksamen Oberfläche der dielektrischen Platte (1) angeordnet sind.

2. Vorrichtung zum Schutz vor Energieaussetzung nach Anspruch 1, **dadurch gekennzeichnet, dass** die dielektrische Platte (1) mit einer dielektrischen Materialschicht (40) beschichtet ist.

## Revendications

1. Dispositif de protection contre l'exposition à l'énergie comprenant une plaque diélectrique (1), une surface efficace laquelle comprend des applicateurs métalliques de la forme d'une étoile à cinq branches (2), d'une étoile à six branches (3) et d'une étoile à huit branches (4), d'un triangle (5), et d'un polygone en dents de scie à n branches (6) agencé dessus, **caractérisé en ce que** la surface efficace de la plaque diélectrique (1) est pourvue de vingt et un applicateurs métalliques additionnels (7) à (27) de la forme de quadrilatères et douze applicateurs métalliques additionnels (28) à (39) de la forme de triangles tous les applicateurs étant agencés sur la surface efficace de la plaque diélectrique (1) comme cela peut être vu sur la figure 2.

2. Dispositif de protection contre l'exposition à l'énergie selon la revendication 1, **caractérisé en ce que** la plaque diélectrique (1) est revêtue d'une couche de matériau diélectrique (40).
